# EUROPEAN PATENT SPECIFICATION

(11) **EP 0 505 636 B1**
(45) Date of publication and mention of the grant of the patent: **06.03.1996**
(21) Application number: 91306196.6
(22) Date of filing: 08.07.1991
(51) Int. Cl.: G01N 33/558, G01N 33/74, G01N 33/76

(54) **Immunochromatographic assay method**
Immunochromatografisches Nachweisverfahren
Essai immunochromatographique

(30) Priority: 28.03.1991 JP 64504/91
(43) Date of publication of application: 30.09.1992
(73) Proprietor: ROHTO PHARMACEUTICAL CO., LTD., Ikuno-ku, Osaka 544 (JP)
(72) Inventor: Koike, Tetsuo, Matsubara-shi, Osaka (JP)
(74) Representative: Perry, Robert Edward

(56) References cited:
- EP-A- 0 260 965
- EP-A- 0 262 328
- EP-A- 0 299 428

## Description

The present invention relates to a method of determining, by the appearance of a symbol, whether or not a certain ligand to be detected is present in a specimen, by means of an immunochromatographic method which combines the so-called sandwich immunoassay method with chromatography used for separation of reaction products from unreacted components (typically called BF separation). The method of this invention is to be used in the field of in vitro diagnosis, and particularly in the field of diagnosis by visual determination; since the results to be judged are indicated clearly by symbols, a person with no particular training can easily judge the diagnostic results.

According to the known principles of immunochromatography, a first antibody (Ab₁) which binds with specificity to the ligand (L) to be detected is affixed near the centre of a, say, rectangular chromatography medium, e.g. filter paper. A second antibody (Ab₂), namely an antibody different from the first antibody yet which also binds with specificity to the ligand to be detected, is prepared separately and bound to an easily detectable marker (M) substance to prepare a marked second antibody. When this second antibody is mixed with a liquid specimen thought to contain the ligand to be detected, a M-Ab₂-L conjugate is formed. If a liquid specimen containing this conjugate is placed in contact with the edge of the chromatography medium and developed, the conjugate will move gradually in the direction of development and reach the affixed first antibody, thus forming a M-Ab₂-L-Ab₁ complex. In this manner, a coloured mark or other symbol will be left by the marker at the site at which the first antibody was affixed; it is thereby possible to confirm the presence of the ligand to be detected.

Many in vitro diagnostic kits based on immunochromatography are known and available commercially; in most of these, the method of determining the result is based on detecting the presence or absence of a spot or line at the site of the first antibody. When results are determined in this way, it is possible that the positive or negative result may be ambiguous depending on the intensity of the tinting.

To solve this problem, a (+) or (-) indicator used to simplify the judging of results by the user has been proposed (in Japanese Patent Application Public Disclosure No. Sho 64-32169). In this method, a symbol (+) is placed on the chromatography medium and the first antibody is bound to one line, e.g. the vertical line, of the symbol, while a sample of the ligand to be detected or antibody to the second antibody (Ab_{2Ab}) is bound to the horizontal line. If the liquid specimen does not contain the ligand to be detected, a M-Ab₂-L or M-Ab₂-Ab_{2Ab} complex is formed only on the horizontal line, and the symbol (-) appears as the result. If the liquid specimen does contain the ligand to be detected, one of the complexes will be formed on the horizontal line of the (+) symbol, while a M-Ab₂-L-Ab₁ complex is formed on the vertical line, so the symbol (+) appears.

Using this method, even in the event that the sample of the ligand to be detected and/or the second antibody bound to the marker affixed to the chromatography medium were to become inactive for some reason, or if a major error in handling were to occur, the horizontal line will not appear, so it is possible to determine whether the diagnostic test was performed correctly or not. In this manner, this (+) or (-) indicator method has the great advantage that the validity of the diagnostic test can be verified.

This (+) or (-) indicator method nevertheless has a major drawback in that it is difficult to get a clear colour indication in the line drawn parallel to the direction of development, namely the vertical line of the (+) symbol. This is because, if the liquid specimen contains the ligand to be detected, when the M-Ab₂-L conjugate reaches the lower edge of the vertical line of the (+) symbol and reacts with the first antibody, a M-Ab₂-L-Ab₁ complex is formed; however, the complex formed on the lower portion of this line impedes the migration of the liquid specimen so the liquid specimen, does not come into contact with the portion of the affixed first antibody on the upper part of the vertical line. As is evident from the accompanying drawings, this may result in only the portion of the line lower than the horizontal line reacting or only the top edge and bottom edge of the vertical line reacting, so a true (+) symbol is not formed correctly.

For these reasons, the currently-available diagnostic kits based on the (+) or (-) indicator method usually indicate that even an incomplete (+) symbol with a missing upper edge or central portion should be considered to be positive.

According to one solution to this problem, the line parallel to the direction of development (the vertical line) is pre-printed with the same colour as the marker, so that a "+" is indicated when a reaction occurs in the line perpendicular to the direction of development (the horizontal line). An example of this is the commercially-available C.A.R.D.S±®O.S kit made by Pacific Biotech, Inc.

In another solution, the horizontal and vertical lines are oblique to the direction of development. An example is the commercially-available TESTPACK+Plus kit made by Abbott Laboratories, Inc.

However, these methods have various drawbacks and are not satisfactory. In the first case, the means of verifying the validity of the diagnostic test, namely the method of binding a sample of the ligand to be detected to the horizontal line, cannot be employed, so the reliability of the diagnostic test cannot be guaranteed. In the second case, the reaction merely occurs in a skewed "V" shape, so that the given drawbacks are not overcome and a true (+) symbol will still not be formed correctly.

According to the present invention, the passage of the liquid specimen is disrupted. By this extremely simple expedient, the first antibody on the vertical line of the (+) symbol is allowed to react completely. It has also been found that, by changing the (+) or (-) indicator to other symbols, e.g. the letters "Y" and "N" which can indicate YES and NO, similar clarity can also be obtained.

The liquid specimen to be tested is, for example, a body fluid such as blood or urine, or a dilute solution thereof, and which contains, or is suspected to contain, the substance or ligand to be detected. The ligand to be detected contributes to an immunoreaction, and is particularly an antigenic substance which is subject to detection in various types of diagnosis, namely haptens and antibodies. Examples of antigenic substances which may be the subject of detection include thyroid-stimulating hormone (TSH), follicle-stimulating hormone (FSH), human chorionic gonadotropin (hCG) and luteinizing hormone (LH). Haptens that may be the subject of detection in the present invention include estrone, estradiol, testosterone, progesterone, and their ester derivatives.

The chromatography medium may be a carrier of the type normally used in chromatography, such as glass filters, nitrocellulose, nylon or filter paper. As the marker, colloidal gold particles, coloured latex, insoluble dye polymers or another coloured substance is preferred, but a colourless substance which becomes coloured when put into contact with a colouring reagent may also be used.

The "developer" to be obstructed comprises the liquid specimen and the second antibody. The means for disrupting its flow is, for example, a notch or notches provided in the chromatography medium, or a hydrophobic substance applied to the medium. The site or sites at which this means of obstruction is provided is not critical, but is preferably upstream of the site at which the first antibody is affixed, and one or more lateral notches, e.g. at the edges of the chromatography medium, are particularly preferable. There are cases in which the provision of a downstream site as well is preferred.

There are no particular limitations as to the shape of suitable notches; various shapes such as rectangles, half-circles, triangles, and approximate triangles are all effective. Again, when applying a hydrophobic substance, various shapes can be employed.

The hydrophobic substance is any substance which adheres to the chromatography medium, including hydrophilic polymers and similar materials which may be hydrophilic when applied but then become hydrophobic during subsequent polymerization or the like. One specific example is an oil-based ink.

There are many known methods, direct and indirect, of affixing the first antibody to the chromatography medium. Direct methods include (1) the use of bromine cyanide, glutaraldehyde or carbodiimide to affix the antibodies with covalent bonds (the covalent bond method) and (2) physical adsorption (the non-covalent bond method). Indirect methods include binding the antibody reagent to latex particles or other insoluble particles, and then affixing the particles, so either covalent bond methods or non-covalent bond methods can be used. In this case, the size of the latex particles must be selected such that they will adhere sufficiently to the chromatography medium but not move during chromatography development.

The marked second antibody may be prepared by mixing a freeze-dried extract with the liquid specimen at the time when the diagnostic test is to be performed. Alternatively, a non-woven fabric or other porous polymer may be impregnated with the marked second antibody, freeze-dried, and then secured to a site upstream of the site on the chromatography medium at which the first antibody is affixed. In this case, the person performing the test need only apply the liquid specimen to the end of the chromatography medium, eliminating the work of mixing the marked second antibody and the liquid specimen. More specifically, the marked second antibody, secured in the direction of development of the liquid specimen, is eluted by the liquid specimen during development, thus forming the conjugate M-Ab₂-L which is transported to the affixed first antibody.

While the present invention has been described above based on the sandwich method, comprising the formation of [first antibody]-[ligand to be detected (antigen)]-[second antibody], those skilled in the art can readily understand that the present invention can also be implemented in a system made up of [antigen]-[ligand to be detected (antibody)]-[antibody to the ligand to be detected]. Therefore, the present invention is not limited to the case in which the ligand to be detected is an antigen, but also includes the case in which it is an antibody, so the present invention may be embodied in this case as if each instance of antibody were replaced by antigen, and vice versa.

The following Examples illustrate the invention, the results of which are shown in the accompanying drawings. Each drawing shows symbols and notches, and the observed effect at different concentrations of hCG (mIU/ml).

### Example 1

### (1) Preparation of solid-phase latex A

A commercial latex emulsion (N-450, made by Sekisui Chemical Co., Ltd.) is diluted to a solids concentration of 0.6 wt.% in phosphate-buffered saline (PBS) solution, 1 ml of which is collected in an Eppendorf® micro-centrifuge with 1 ml of rabbit antibody to human chorionic gonadotropin (hCG) (600 µg/ml concentration) and dissolved by shaking for 2 hours at room temperature to bind the rabbit antibody to the latex particles. The latex particles are then centrifuged and washed three times in PBS containing 0.1 wt.% bovine serum albumin (BSA) and then suspended again in PBS to give a final volume of 2 ml, to give solid-phase latex A.

### (2) Preparation of solid-phase latex B

Solid-phase latex B is prepared in the same manner as solid-phase latex A, but using hCG (made by Teikoku Hormone Mfg. Co., Ltd.) instead of the rabbit anti-hCG antibody.

### (3) Preparation of colloidal gold particles

After boiling 200 ml of a 0.01 wt.% aqueous solution of gold chloride, a 1 wt.% aqueous solution of sodium citrate is added and the mixture boiled while heating until the colour of the solution changes from a light yellow to purple or red, to give a disperse solution of colloidal gold with an average particle size of 0.03 µm.

### (4) Preparation of colloidal gold marker antibodies

A solution of potassium carbonate is added to the disperse solution of colloidal gold which has a gold concentration of 0.01 wt.% until a pH of 7.6 is reached. To this is added a quantity of monoclonal antibodies to hCG (obtained by means of the well-known hybridoma method, prepared from mouse ascites or cell-culture supernate) sufficient to obtain a proportion of 10 µg per ml of disperse solution of colloidal gold, of which 10 ml is added to 0.1 ml of 30 wt.% BSA. The mixture is centrifuged, the supernate removed, and PBS containing 0.1 wt.% BSA is used to centrifuge and wash the mixture three times. The mixture is dispersed back into 10 ml of the aforesaid PBS, and 0.25 ml is decanted into a test tube (method A).

A 12x12 mm square of Benrize® non-woven fabric (made by Asahi Chemical Industry Co., Ltd.) is impregnated with 0.25 ml of a disperse solution of the colloidal gold marker antibodies and freeze-dried to prepare a non-woven cloth containing a colloidal gold marker antibody (method B).

### (5) Preparation of chromatography strips A

A commercial glass filter (GC-50, made by Toyo Roshi Co.) is cut into 12x60 mm thin strips which are placed upon glass plates (TLC plates) and secured with tape for ease of handling. At a site 30 mm from the bottom edge of the strips, an Acurajetter® liquid jet device (made by Nordson Co.) and an XY table aerostage (made by THK Co.) are used to jet-print the solid-phase latex A at a rate of 0.1 µm/mm for a length of 10 mm in the direction of development, namely the lengthwise direction of the strip. After drying, solid-phase latex B is jet-printed at a rate of 0.1 µm/mm for a length of 10 mm perpendicular to the direction of development, namely across the short direction of the strip, thereby printing a "+".

### (6) Preparation of an hCG-containing specimen

An injectable type of hCG ("Gonatropin 5000", made by Teikoku Hormone Mfg. Co., Ltd.) is diluted with PBS containing 0.1 wt.% BSA to prepare hCG-containing specimens with hCG concentrations of 25, 50 and 100 mIU/ml.

### (7) Immunochromatography

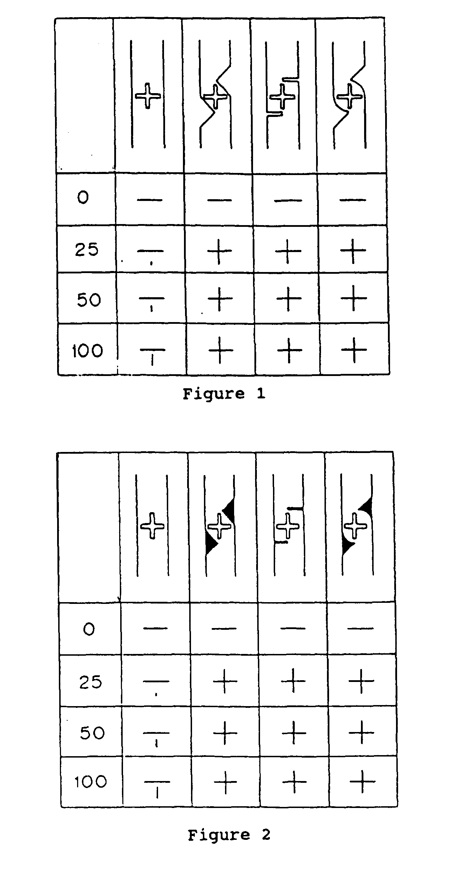

Various notches are cut around the reaction site (the site at which the first antibody is affixed) on the chromatography strips (see Figure 1), so that the mixture of the liquid specimen and colloidal gold marker antibodies cannot develop in the notched portions.

One 500 µl portion of each of the aforesaid hCG-containing specimens or a blank of 500 µl of PBS containing 0.1 wt.% BSA is added each to a test tube containing the colloidal gold marker antibodies and stirred. The mixtures thus obtained are allowed to impregnate only the lower 5 mm of the chromatography strips with various notches cut in, or a control chromatography strip with no notches, and the liquid specimen is allowed to migrate. After 5 minutes have passed, the "+" or "-" indicators in the reaction sites are compared visually.

The results of the indicator appearances and shapes for this two-step immunochromatography on notched chromatography strips are illustrated in Figure 1.

### Example 2

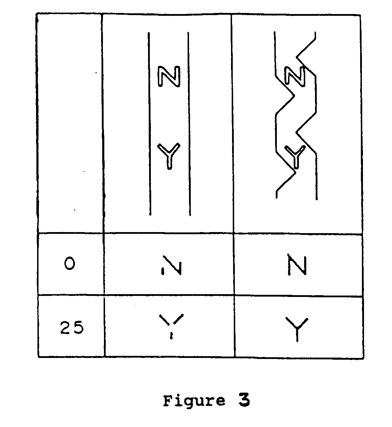

Commercial oil-based ink (correction fluid, made by Zebra Co., Ltd.) is applied in various patterns around the reaction site on the same chromatography strips as in Example 1 (see Figure 2). After drying, the same mixtures of liquid specimen and colloidal gold marker antibodies as in step (7) of Example 1 are allowed to impregnate the strips. After 5 minutes of development, the "+" or "-" indicators in the reaction sites are compared visually.

The results of the indicator appearances and shapes for this two-step immunochromatography on notched chromatography strips, to which an oil-based ink is applied, are illustrated in Figure 2.

### Example 3

Portions of the chromatography strips prepared in Example 1 are cut between 10 mm and 15 mm from the bottom edge, and these portions are connected by staples with the non-woven cloth containing a colloidal gold marker antibody, to give a strip which again has an overall length of 60 mm.

Various notches are cut, as in step (7) of Example 1, on the immunochromatography strips (see Figure 3), and 500 µl portions of each of the hCG-containing specimens as in step (7) of Example 1 or a blank of PBS containing 0.1 wt.% BSA in test tubes are allowed to impregnate only the lower 5 mm of the strips. After 5 minutes of development, the "+" or "-" indicators in the reaction sites are compared visually.

The results of the indicator appearances and shapes of this one-step immunochromatography on notched chromatography strips to which second antibodies are bound, are again as illustrated in Figure 1.

### Example 4

Commercial oil-based ink is applied as in Example 2 to the same chromatography strips as in Example 3. After drying, the same liquid specimens as in Example 3 are allowed to impregnate only the lower 5 mm of the strips. After 5 minutes of development, the "+" or "-" indicators in the reaction sites are compared visually.

The results of the indicator appearances and shapes of this one-step immunochromatography on notched chromatography strips to which second antibodies are bound and to which an oil-based ink is applied, are again as illustrated in Figure 2.

### Example 5

A commercial glass filter is made into 12x80 mm thin strips which are secured to glass plates (TLC plates). The aforesaid printing device is used to print a "Y" with 0.1 µl/mm of solid-phase latex A (see Example 1) at a site 30 mm from the bottom of the strip, and to print an "N" with 0.1 µl/mm of solid-phase latex B (see Example 1) at a site 50 mm from the bottom of the strip.

Notches are cut around the sites at which the "Y" or "N" indicators are printed on the above immunochromatography strips (see Figure 3). 500 µl portions of each of the hCG-containing specimens as in step (7) of Example 1 or a blank of PBS containing 0.1 wt.% BSA in test tubes are allowed to impregnate only the lower 5 mm of the strips. After 10 minutes of development, the "Y" or "N" indicators in the reaction sites are compared visually.

The results of the indicator appearances and shapes of this two-step immunochromatography on notched chromatography strips, are illustrated in Figure 3.

As is evident from Figures 1 to 3, if the means of obstructing the passage of developer according to the method of this invention is provided on the chromatography medium, not only the symbol (+) but also complex symbols such as (Y) and (N) appear clearly.

## Claims

1. An immunochromatographic method in which a first antibody which bonds with specificity to a ligand to be detected in a liquid specimen is affixed in the shape of a symbol on a chromatography medium, the liquid specimen is allowed to pass through the medium in or into contact with a marked second antibody which bonds with specificity to the ligand, such that the presence or absence of the ligand in the liquid specimen can be determined by the symbol which can be visually detected due to the marker; characterised in that the passage of the liquid specimen is obstructed immediately before, and optionally also immediately after, the affixed first antibody.

2. The method of claim 1, which comprises the prior step of mixing the liquid specimen with the marked second antibody.

3. The method of claim 1, in which the liquid specimen comes into contact, during its passage before the first antibody, with the second antibody which is bonded.

4. The method of any preceding claim, in which the chromatography medium is selected from glass fibres, nitrocellulose and nylon.

5. The method of any preceding claim, in which the marker is selected from colloidal gold particles, coloured latex and insoluble dye polymers.

6. The method of any preceding claim, in which the first antibody has been affixed to the medium by direct printing with an aqueous solution of the first antibody.

7. The method of any of claims 1 to 5, in which the first antibody has been affixed to the medium by printing with latex to which the first antibody is bonded.

8. The method of any preceding claim, in which the means of obstruction comprises one or more lateral notches.

9. The method of any of claims 1 to 7, in which the means of obstruction comprises a hydrophobic substance applied to the medium.

10. The method of any preceding claim, in which the means of obstruction defines liquid flow past the symbol.

11. A chromatography medium to which an antibody is affixed in the shape of a symbol, additionally comprising an obstruction to liquid flow through the medium past the symbol, the obstruction being immediately before, and optionally also immediately after, the symbol.

12. The medium of claim 11, in which the material of said medium is as defined in claim 4.

13. The medium of claim 11 or claim 12, in which the obstruction is as defined in any of claims 8 to 10.

## Patentansprüche

1. Immunchromatographisches Verfahren, bei dem ein erster Antikörper, der sich spezifisch an einen in einer flüssigen Probe nachzuweisenden Liganden bindet, in Form eines Symbols an ein Chromatographiemedium gebunden wird, und man die flüssige Probe sich durch ein Medium bewegen läßt, wobei diese mit einem markierten zweiten Antikörper, der sich spezifisch an den Liganden bindet, in Kontakt steht oder in Kontakt kommt, so daß das Vorhandensein oder die Abwesenheit des Liganden in der flüssigen Probe mittels des Symbols bestimmt werden kann, das aufgrund der Markersubstanz durch Augenschein wahrgenommen werden kann; dadurch gekennzeichnet, daß der Durchgang der flüssigen Probe unmittelbar vor und gegebenenfalls auch unmittelbar hinter dem gebundenen ersten Antikörper blockiert wird.

2. Verfahren nach Anspruch 1, welches den Anfangsschritt des Mischens der flüssigen Probe mit dem markierten zweiten Antikörper umfaßt.

3. Verfahren nach Anspruch 1, bei dem die flüssige Probe während ihres Durchgangs vor dem ersten Antikörper mit dem zweiten Antikörper in Kontakt kommt, der gebunden wird.

4. Verfahren nach einem der vorhergehenden Ansprüche, bei dem das Chromatographiemedium aus Glasfasern, Nitrocellulose und Nylon ausgewählt wird.

5. Verfahren nach einem der vorhergehenden Ansprüche, bei dem die Markersubstanz aus kolloiden Goldteilchen, farbigem Latex und unlöslichen Farbstoffpolymeren ausgewählt wird.

6. Verfahren nach einem der vorhergehenden Ansprüche, bei dem der erste Antikörper durch direktes Aufdrucken einer wäßrigen Lösung des ersten Antikörpers an das Medium gebunden worden ist.

7. Verfahren nach einem der Ansprüche 1 bis 5, bei dem der erste Antikörper durch Bedrucken mit Latex, an welchen der erste Antikörper gebunden ist, an das Medium gebunden worden ist.

8. Verfahren nach einem der vorhergehenden Ansprüche, bei dem die Blockade (Hemmung) die Anwednung einer oder mehrerer seitlichen Kerben umfaßt.

9. Verfahren nach einem der Ansprüche 1 bis 7, bei dem die Blockade (Hemmung) die Anwendung einer auf das Medium aufgebrachten hydrophoben Substanz umfaßt.

10. Verfahren nach einem der vorhergehenden Ansprüche, bei dem die Blockade (Hemmung) den Flüssigkeitsfluß über das Symbol hinaus begrenzt.

11. Chromatographiemedium, an welches ein Antikörper in der Form eines Symbols gebunden ist und welches zusätzlich eine Blockierung des Flüssigkeitsflusses im Medium über das Symbol hinaus umfaßt, wobei sich die Blockierung unmittelbar vor und gegebenenfalls auch unmittelbar hinter dem Symbol befindet.

12. Medium nach Anspruch 11, dessen Material wie in Anspruch 4 definiert ist.

13. Medium nach Anspruch 11 oder 12, bei dem die Blockierung wie in einem der Ansprüche 8 bis 10 definiert durchgeführt wird.

## Revendications

1. Procédé immunochromatographique dans lequel un premier anticorps qui se lie de manière spécifique à un ligand à détecter dans un échantillon liquide est fixé sous la forme d'un pictogramme sur un support de chromatographie, l'échantillon liquide est amené à passer à travers le support ou en contact avec un second anticorps marqué qui se lie de manière spécifique au ligand, de sorte que la présence ou l'absence du ligand dans l'échantillon de liquide puisse être déterminée par le pictogramme qui peut être visuellement détecté grâce au marqueur; caractérisé en ce que le passage de l'échantillon liquide est entravé immédiatement avant, et facultativement également immédiatement après, le premier anticorps fixé.

2. Procédé selon la revendication 1, qui comporte l'étape préalable de mélange de l'échantillon liquide avec le second anticorps marqué.

3. Procédé selon la revendication 1, dans lequel, pendant son passage avant le premier anticorps, l'échantillon liquide vient en contact avec le second anticorps qui est lié.

4. Procédé selon l'une quelconque des revendications précédentes, dans lequel le support de chromatographie est choisi parmi fibres de verre, nitrocellulose et nylon.

5. Procédé selon l'une quelconque des revendications précédentes, dans lequel le marqueur est choisi parmi des particules d'or colloïdal, du latex coloré et des colorants polymères insolubles.

6. Procédé selon l'une quelconque des revendications précédentes, dans lequel le premier anticorps a été fixé sur le support par impression directe avec une solution aqueuse du premier anticorps.

7. Procédé selon l'une quelconque des revendications 1 à 5, dans lequel le premier anticorps a été fixé sur le support par impression avec un latex auquel le premier anticorps a été lié.

8. Procédé selon l'une quelconque des revendications précédentes, dans lequel le moyen d'entrave comporte une ou plusieurs encoches latérales.

9. Procédé selon l'une quelconque des revendications 1 à 7, dans lequel le moyen d'entrave comporte une substance hydrophobe appliquée sur le support.

10. Procédé selon l'une quelconque des revendications précédentes, dans lequel le moyen d'entrave définit un écoulement liquide au-delà du pictogramme.

11. Support de chromatographie sur lequel un anticorps a été fixé sous la forme d'un pictogramme, comportant de plus une entrave à l'écoulement liquide à travers le support au-delà du pictogramme, l'entrave étant située immédiatement avant, et facultativement également immédiatement après le pictogramme.

12. Support selon la revendication 11, dans lequel le matériau dudit support est défini en revendication 4.

13. Support selon la revendication 11 ou la revendication 12, dans lequel l'entrave est telle que défini dans l'une quelconque des revendications 8 à 10.
